Europäisches Patentamt

European Patent Office

Office européen des brevets

(11) Veröffentlichungsnummer : **0 399 341 B1**

(12) **EUROPÄISCHE PATENTSCHRIFT**

(45) Veröffentlichungstag der Patentschrift :
**14.04.93 Patentblatt 93/15**

(51) Int. Cl.⁵ : **A23L 1/305, A61K 37/18**

(21) Anmeldenummer : **90109185.0**

(22) Anmeldetag : **16.05.90**

(54) **Wässrige Zusammensetzung für die parenterale Ernährung.**

(30) Priorität : **24.05.89 DE 3916903**

(43) Veröffentlichungstag der Anmeldung :
**28.11.90 Patentblatt 90/48**

(45) Bekanntmachung des Hinweises auf die
Patenterteilung :
**14.04.93 Patentblatt 93/15**

(84) Benannte Vertragsstaaten :
**AT BE CH DE DK FR GB IT LI NL SE**

(56) Entgegenhaltungen :
**EP-A- 0 013 962**
**EP-A- 0 148 680**
**EP-A- 0 232 652**
**WO-A-88/01872**
**DE-A- 2 946 563**
**PATENT ABSTRACTS OF JAPAN, Band 8, Nr.**
**102 (C-222)(1539), 12. Mai 1984**

(73) Patentinhaber : **LEOPOLD PHARMA
GESELLSCHAFT m.b.H.
Hafnerstrasse 36
A-8055 Graz (AT)**

(72) Erfinder : **Groke, Karl, Dr.
Nr. 327
A-8063 Eggersdorf (AT)**
Erfinder : **Musil, Horst-Eberhard, Dr.
Hans-Riehl-Gasse 8
A-8043 Graz (AT)**
Erfinder : **Pitter, Johannes
Nr. 112
A-8120 Peggau (AT)**

(74) Vertreter : **Dr. Fuchs, Dr. Luderschmidt Dr.
Mehler, Dipl.-Ing. Weiss Patentanwälte
Abraham-Lincoln-Strasse 7, Postfach 4660
W-6200 Wiesbaden (DE)**

## Beschreibung

Die Erfindung betrifft eine wäßrige Zusammensetzung auf Basis von essentiellen und nicht essentiellen Aminosäuren, die eine auf die Normalpatienten zugeschnittene vollwertige Versorgung mit Aminosäuren bei möglichst geringer Wasserzufuhr gestattet.

Es ist sei langem üblich, zur parenteralen Ernährung neben energieliefernden Bestandteilen Aminosäuremischungen heranzuziehen, wobei die Verabreichung von Gemischen reiner, synthetischer L-Aminosäuren bevorzugt ist. Dabei wurde bisher die Meinung vertreten, daß die Aminosäuren in wäßriger Lösung nur in Konzentrationen bis zu 10 Gew.% verwendet werden können, da Lösungen höherer Konzentrationen zum Auskristallisieren neigen (Helwig, Moderne Arzneimittel, Oktober 1988, 35, 4, Aminosäurelösungen, Seite 37). Ferner geben sie auch zu Verfärbungen Anlaß.

In der WO-A 88/01872 wird eine parenteral zu verabreichende Lösung zur Behandlung von Atherosklerose beschrieben, die neben den wirksamen Bestandteilen Leucin, Isoleucin, Valin und Arginin aus Ernährungsgründen vorzugsweise auch die übrigen essentiellen Aminosäuren, Alanin und Histidin sowie gegebenenfalls zusätzlich eine oder mehrere der Aminosäuren Prolin, Serin, Tyrosin, Glycin, Glutaminsäure, Asparaginsäure und Cystein enthalten sollen. Für diese bevorzugten Lösungen, deren Prozentgehalt an den einzelnen Aminosäuren bezogen auf das Gewicht des Gesamtaminosäuregemisches im Hinblick auf die Wahlmöglichkeit von 11 bis 18 Aminosäuren naturgemäß in sehr weiten Grenzen schwanken kann, wird angegeben, daß sie mindestens 4 g, vorzugsweise 7,5 bis 12 g an der Gesamtaminosäuremischung pro 100 ml Lösung enthalten, wobei als oberste Konzentrationsgrenze die Löslichkeitsgrenze des verwendeten Aminosäuregemisches genannt wird, die manchmal sogar 20 % Gewicht pro Volumen betragen könne. Eine konkrete Angabe über Aminosäuregemische, bei denen diese Konzentration von 20 % Gewicht pro Volumen tatsächlich erreicht werden könnte, wird nicht gemacht.

In den Patents-Abstracts of Japan, Band 8, Nr. 102 (C-222) (1539) ist eine Aminosäurenlösung zur Ernährung von Patienten mit verminderter Leberfunktion beschrieben, die neben den essentiellen Aminosäuren noch 10 nichtessentielle Aminosäuren enthält. In dieser Lösung, die das Aminosäurengemisch in einer Konzentration von 2 - 15 Gew.-%, vorzugsweise 3 - 12 Gew.-%, enthalten soll, beträgt der Anteil der verzweigtkettigen Aminosäuren im Gemisch 26 - 34 Gew.-%. Das Gewichtsverhältnis von L-Tyrosin zu L-Phenylalanin ist mit 1 : (12 bis 17), jenes von L-Cystein zu L-Methionin bei einem Cysteingehalt von 1,26 - 1,54 g pro 100 g Aminosäurengemisch mit 1 : (2,0 bis 3,1) festgesetzt.

Die aus der DE-A 2.946.563 bekannten Aminosäurenlösungen, die ebenfalls für die Verabreichung an Patienten mit Leberinsuffizienz bestimmt sind und die dieselben Aminosäuren wie die Lösungen gemäß dem oben zitierten Patents Abstract of Japan sowie zusätzlich Ornithin enthalten, weisen ebenfalls im Hinblick auf die spezielle Indikation einen hohen Gehalt an verzweigtkettigen Aminosäuren von rund 30 Gew.-% und einen Cysteingehalt von 2,0 ± 1,0 Gewichtsteilen pro 100 Gewichtsteilen Aminosäurengemisch auf. Die Konzentration dieser Aminosäurenlösung bleibt dabei völlig unerwähnt, es wird lediglich ausgesagt, daß die Aminosäuren in den angegebenen Mengen durch Mischen in Wasser oder durch Auflösen der einzelnen Aminosäuren in Wasser und anschließendes Mischen der erhaltenen Lösungen zu Aminosäurenlösungen der angegebenen Zusammensetzung der Aminosäurekomponente verarbeitet werden.

In der EP-B 0 013 962 wird eine Aminosäuren und Mineralsalze enthaltende, glukosefreie Infusionslösung beschrieben, die 10 bis 200 g essentielle und nichtessentielle Aminosäuren sowie Kinine in einer Menge von 50 bis 10.000 µg je Liter Lösung enthält. Die als Beispiel für eine Lösung mit einem Aminosäurengehalt von 200 g/l genannte Lösung C, besitzt einen Anteil von 52 g an essentiellen Aminosäuren und 148 g an nichtessentiellen Aminosäuren, das entspricht einem Gehalt an essentiellem Stickstoff von 5,96 g und an nichtessentiellem Stickstoff von 23,66 g. Das Verhältnis $N_{essentiell}$ zu $N_{nonessentiell}$ beträgt somit 1:3,97, liegt also weit außerhalb der empfohlenen Grenzen von 1:1 und 1:3. An nichtessentiellen Aminosäuren sind außer den vier semiessentiellen Aminosäuren Arginin, Alanin, Histidin und Prolin nur noch die leichtlöslichen Aminosäuren Glycin und L-Glutaminsäure zugegen, und zwar in einer Menge von 36 bzw. 38 g/l. Um von dieser Lösung 100 g Aminosäuren pro Tag verabreichen zu können, muß die empfohlene Obergrenze der täglichen Gabe an Glycin und L-Glutaminsäure von je 14 g/Tag in beiden Fällen erheblich überschritten werden, was zu Aminosäureimbalanzen und Nebenwirkungen Anlaß gibt.

Aus der EP-A 0 182 356 sind Zusammensetzungen für die intravenöse, orale oder intragastrointestinale Verabreichung bekannt geworden, in denen die Aminosäurekomponente eine Konzentration von bis zu 40 Gew.% in wäßriger Lösung besitzen kann. In diesen Zusammensetzungen besteht diese Aminosäurenkomponente aber nur zum Teil aus reinen Aminosäuren. Ein wesentlicher Teil der Aminosäurenkomponente, nämlich 0,2 bis 30 Gew.% der gesamten wäßrigen Zusammensetzung, liegen in Form von leichtlöslichen Di- oder Tripeptiden vor, die teilweise Glycin, zum anderen Teil Alanin, Lysin oder Arginin als N-terminale Gruppe besitzen. Bevorzugt beträgt der Anteil der Di- oder Tripeptide 2 bis 20 Gew.% der gesamten wäßrigen Zusammensetzung. Diese Zusammensetzungen bieten aufgrund der hohen Konzentration eine hohe Dosierungsmöglichkeit an Proteinbaustoffen bei möglichst geringer Wasserbelastung der Patienten, was besonders bei Patienten mit Herzinsuffizienz oder Nierenstörungen von Bedeutung ist.

Ein Nachteil dieser Nährzusammensetzungen ist, daß zu ihrer Bereitung synthetisch gewonnene Di- oder Tripeptide herangezogen werden müssen, die über mehrstufige Verfahren aus reinen, synthetischen Aminosäuren erzeugt werden müssen. Das bedeutet einen großen Arbeitsaufwand für die Herstellung solcher Lösungen und damit eine hohe wirtschaftliche Belastung für die künstliche Ernährung von Patienten. Bei der parenteralen Verabreichung solcher Lösungen kommt als weiterer Nachteil hinzu, daß mit dem Gehalt an Di- oder Tripeptiden die Gefahr des Vorhandenseins oder die Bildung von unerwünschten Nebenprodukten wächst, so daß kürzere Lagerzeiten die Folge sind, sowie daß Komplikationen als Folge des Sterilisation auftreten können.

Überraschenderweise wurde nun gefunden, daß es möglich ist, unter alleiniger Verwendung von reinen kristallinen Aminosäuren wäßrige, für die parenterale Verabreichung geeignete Nährlösungen mit einer Aminosäurekonzentrationen von 15 bis 23 und einem Verhältnis $N_{essentiell}$ zu $N_{nonessentiell}$ im Bereich von 1:1 bis 1:3 Gew.% zu schaffen, die ein vollwertiges Aminosäuremuster aufweisen und trotzdem eine ausgezeichnete Stabilität besitzen.

Dies ist entgegen der bisherigen Lehrmeinung dann möglich, wenn ganz bestimmte Werte hinsichtlich der Zusammensetzung des Aminosäuregemisches eingehalten werden. Diese lassen sich weder aus der Lehre der WO-A 88/01872 noch aus Patents Abstract of Japan, Band 8, Nr. 102 (C-222) (1539) oder aus der DE-A 2.946.563 herleiten, da sie sich grundlegend von den in diesen Druckschriften angegebenen Gewichtsverhältnissen der einzelnen Aminosäuren im Gemisch unterscheiden und den beiden letztgenannten Druckschriften auch keinerlei Andeutungen bezüglich einer Möglichkeit der Herstellung von Aminosäurenlösungen in einer Konzentration von 15 - 23 Gew.-% zu entnehmen ist. Vielmehr wird 15 Gew.-% als oberste Konzentrationsgrenze einer Aminosäurenlösung angegeben, die zwar aus etwa denselben Aminosäuren, jedoch in wesentlich anderer quantitativer Zusammensetzung besteht.

Gegenstand der vorliegenden Erfindung ist demnach eine wäßrige Zusammensetzung für die parenterale Ernährung, enthaltend die essentiellen und nicht essentiellen Aminosäuren, in einem Verhältnis $N_{essentiell}$ zu $N_{nonessentiell}$ von 1:1 bis 1:3, dadurch gekennzeichnet, daß sie als Aminosäurenkomponente nur freie Aminosäuren in einer Konzentration von 15 bis 23 Gew.% aufweist und sich diese je 100 g aus

| L-Isoleucin | 2,6 - 3,2 g |
| L-Leucin | 6,0 - 7,5 g |
| L-Lysin | 6,5 - 8,0 g |
| L-Methionin | 2,6 - 3,2 g |
| L-Phenylalanin | 4,0 - 5,0 g |
| L-Threonin | 3,0 - 5,0 g |
| L-Tryptophan | 0,8 - 1,2 g |
| L-Valin | 5,0 - 6,0 g |
| L-Arginin | 10,0 - 12,5 g |
| L-Histidin | 1,8 - 3,0 g |
| L-Alanin | 10,0 - 16,0 g |
| L-Asparaginsäure | 5,0 - 6,5 g |
| L-Glutaminsäure | 8,5 - 12,0 g |
| Glycin | 11,5 - 14,0 g |
| L-Prolin | 5,5 - 8,0 g |
| L-Serin | 3,5 - 5,0 g |
| L-Tyrosin | 0,2 - 0,4 g |
| L-Ornithin | 0,1 - 3,0 g |

zusammensetzt.

Der Ausdruck "nur freie Aminosäuren" ist im Sinne der vorliegenden Erfindung so zu verstehen, daß in der Zusammensetzung keine der Aminosäuren als Derivat mit substituierter alpha-Aminogruppe, wie insbesondere als Oligopeptid oder anderes Acylderivat, vorliegt.

Besonders bevorzugt ist hierbei eine Nährlösung mit einer Aminosäurenkonzentration von 20 Gew.% mit folgender Zusammensetzung des Aminosäuregemisches:

| L-Isoleucin | 5,80 g/l |
| L-Leucin | 14,00 g/l |
| L-Lysin | 14,00 g/l |
| L-Methionin | 6,00 g/l |
| L-Phenylalanin | 9,00 g/l |

| | |
|---|---|
| L-Threonin | 7,00 g/l |
| L-Tryptophan | 2,00 g/l |
| L-Valin | 11,00 g/l |
| L-Arginin | 24,00 g/l |
| L-Histidin | 4,00 g/l |
| L-Alanin | 25,00 g/l |
| L-Asparaginsäure | 12,00 g/l |
| L-Glutaminsäure | 20,00 g/l |
| Glycin | 25,40 g/l |
| L-Prolin | 11,80 g/l |
| L-Serin | 8,00 g/l |
| L-Ornithin | 0,40 g/l |
| L-Tyrosin | 0,60 g/l |

Der erfindungsgemäßen Lösung können auch weitere Nährstoffe wie Glucoseersatzstoffe, insbesondere Sorbit oder Xylit, Elektrolyte, Spurenelemente, Vitamine und andere übliche Zusätze von Gesamtnährlösungen einverleibt werden. Auch der Zusatz von Fett ist möglich, wobei es empfehlenswert ist, neben den üblichen Phosphatiden als Emulgator Ubidecarenon als Hilfsemulgator einzusetzen, vor allem dann, wenn auch Elektrolyte zugegen sind.

Die Herstellung der erfindungsgemäßen Lösung erfolgt auf übliche Weise, vorzugsweise unter Stickstoffbegasung. Auch bei der Sterilisation sind die üblichen Methoden einzuhalten, die sich vor allem an der Natur der Kohlenhydratkomponente orientieren. Werden beispielsweise Zuckeralkohole als Kohlenhydratkomponente eingesetzt, so ist z.B. eine Sterilisation bei 120°C möglich und empfehlenswert.

Die erfindungsgemäße Lösung ist vor allem für die parenterale Ernährung von Normalpatienten, das heißt Patienten ohne wesentliche Stoffwechselstörungen, vorgesehen. Wegen des geringen Wassergehaltes empfiehlt sich deren Verwendung aber speziell für Herzkranke und Patienten mit Niereninsuffizienz.

Beispiel 1

| | |
|---|---|
| L-Isoleucin | 5,80 g |
| L-Leucin | 14,00 g |
| L-Lysin.HCl | 17,49 g |
| L-Methionin | 6,00 g |
| L-Phenylalanin | 9,00 g |
| L-Threonin | 7,00 g |
| L-Tryptophan | 2,00 g |
| L-Valin | 11,00 g |
| L-Arginin | 24,00 g |
| L-Histidin | 4,00 g |
| L-Alanin | 25,00 g |
| L-Asparaginsäure | 12,00 g |
| L-Glutaminsäure | 20,00 g |
| Glycin | 25,40 g |
| L-Prolin | 11,80 g |
| L-Serin | 8,00 g |
| L-Ornithin.HCl | 0,51 g |
| L-Tyrosin | 0,60 g |

werden bei 80°C in etwas weniger als 1 Liter Aqua ad infundi iniectaebilia gelöst, worauf der pH-Wert, der sich auf 4,56 eingestellt hat, durch Zugabe von 2,9 g NaOH auf etwa 6 eingestellt wird. Nach Auffüllen auf ein Volumen von 1 Liter wird die Lösung 20 min bei 121°C sterilisiert.

Sie ist nach der Sterilisation völlig farblos und klar und zeigt nach nach 6 mondatiger Lagerung bei Raumtemperatur oder 4°C keine Tendenz zur Verfärbung oder Kristallisation. Das Verhältnis $N_{essentiell}$ zu $N_{nonessentiell}$ beträgt 1:2,7.

Beispiel 2

| | |
|---|---|
| L-Isoleucin | 5,80 g |
| L-Leucin | 14,00 g |
| L-Lysin.HCl | 19,98 g |

| | |
|---|---|
| L-Methionin | 6,00 g |
| L-Phenylalanin | 9,00 g |
| L-Threonin | 10,00 g |
| L-Tryptophan | 2,00 g |
| L-Valin | 11,00 g |
| L-Arginin | 24,00 g |
| L-Histidin | 6,00 g |
| L-Alanin | 32,00 g |
| L-Asparaginsäure | 12,00 g |
| L-Glutaminsäure | 20,00 g |
| Glycin | 32,00 g |
| L-Prolin | 16,00 g |
| L-Serin | 10,00 g |
| L-Ornithin.HCl | 3,57 g |
| L-Tyrosin | 0,60 g |

werden, wie in Beispiel 1 beschrieben, in einer wäßrigen Form, zu einem Volumen von 1 l verarbeitet. Das Verhältnis $N_{essentiell}$ zu $N_{nonessentiell}$ beträgt 1:2,96.

Beispiel 3

Das Beispiel 1 wird wiederholt, jedoch werden zusätzlich in der Infusionslösung 70 g Sorbit und 30 g Xylit gelöst.

**Patentansprüche**

1. Wäßrige Zusammensetzung für die parenterale Ernähung, enthaltend die essentiellen und nichtessentielle Aminosäuren in einem Verhältnis $N_{essentiell}$ zu $N_{nonessentiell}$ von 1 : 1 bis 1 : 3, dadurch gekennzeichnet, daß sie als Aminosäurekomponente nur freie Aminosäuren mit unsubstituierter alpha-Aminogruppe in einer Konzentration von 15 - 23 Gew.-% aufweist und sich diese je 100 g aus

| | |
|---|---|
| L-Isoleucin | 2,6 - 3,2 g |
| L-Leucin | 6,0 - 7,5 g |
| L-Lysin | 6,5 - 8,0 g |
| L-Methionin | 2,6 - 3,2 g |
| L-Phenylalanin | 4,0 - 5,0 g |
| L-Threonin | 3,0 - 5,0 g |
| L-Tryptophan | 0,8 - 1,2 g |
| L-Valin | 5,0 - 6,0 g |
| L-Arginin | 10,0 - 12,5 g |
| L-Histidin | 1,8 - 3,0 g |
| L-Alanin | 10,0 - 16,0 g |
| L-Asparaginsäure | 5,0 - 6,5 g |
| L-Glutaminsäure | 8,5 - 12,0 g |
| Glycin | 11,5 - 14,0 g |
| L-Prolin | 5,5 - 8,0 g |
| L-Serin | 3,5 - 5,0 g |
| L-Tyrosin | 0,2 - 0,4 g |
| L-Ornithin | 0,1 - 3,0 g |

zusammensetzt.

2. Wäßrige Zusammensetzung gemäß Anspruch 1, dadurch gekennzeichnet, daß sie eine Aminosäurekonzentration von 20 Gew.% aufweist und sich die Aminosäuren wie folgt zusammensetzen:

| | |
|---|---|
| L-Isoleucin | 5,80 g/l |
| L-Leucin | 14,00 g/l |
| L-Lysin | 14,00 g/l |
| L-Methionin | 6,00 g/l |
| L-Phenylalanin | 9,00 g/l |
| L-Threonin | 7,00 g/l |

| | |
|---|---|
| L-Tryptophan | 2,00 g/l |
| L-Valin | 11,00 g/l |
| L-Arginin | 24,00 g/l |
| L-Histidin | 4,00 g/l |
| L-Alanin | 25,00 g/l |
| L-Asparaginsäure | 12,00 g/l |
| L-Glutaminsäure | 20,00 g/l |
| Glycin | 25,40 g/l |
| L-Prolin | 11,80 g/l |
| L-Serin | 8,00 g/l |
| L-Ornithin | 0,40 g/l |
| L-Tyrosin | 0,60 g/l |

3.   Wäßrige Zusammensetzung nach den Ansprüchen 1 und 2, dadurch gekennzeichnet, daß sie außerdem andere Nährstoffe, wie Glucoseersatzstoffe, Elektrolyte, Spurenelemente, Vitamine und/oder Fett enthält.

**Claims**

1.   Aqueous composition for the parenteral nutrition comprising the essential and non-essential amino acids in a ratio $N_{essential}$ to $N_{non-essential}$ being 1:1 to 1:3, characterized in that there are only free amino acids as an amino acid component having unsubstituted $\alpha$-amino groups in a concentration of 15 to 23 weight-% and in that, per 100 g, said composition is composed of

| | |
|---|---|
| L-Isoleucine | 2,6 - 3,2 g |
| L-Leucine | 6,0 - 7,6 g |
| L-Lysine | 6,5 - 8,0 g |
| L-Methionine | 2,6 - 3,2 g |
| L-Phenylalanine | 4,0 - 5,0 g |
| L-Threonine | 3,0 - 5,0 g |
| L-Tryptophane | 0,8 - 1,2 g |
| L-Valine | 5,0 - 6,0 g |
| L-Arginine | 10,0 - 12,5 g |
| L-Histidine | 1,8 - 3,0 g |
| L-Alanine | 10,0 - 16,0 g |
| L-Aspartic acid | 5,0 - 6,5 g |
| L-Glutamic acid | 8,5 - 12,0 g |
| Glycine | 11,5 - 14,0 g |
| L-Proline | 5,5 - 8,0 g |
| L-Serine | 3,5 - 5,0 g |
| L-Tyrosine | 0,2 - 0,4 g |
| L-Ornithine | 0,1 - 3,0 g |

2.   Aqueous composition according to claim 1, characterized in that it comprises an amino acid concentration of 20 weight-% and in that it is composed of the following amino acids:

| | |
|---|---|
| L-Isoleucine | 5,80 g/l |
| L-Leucine | 14,00 g/l |
| L-Lysine | 14,00 g/l |
| L-Methionine | 6,00 g/l |
| L-Phenylalanine | 9,00 g/l |
| L-Threonine | 7,00 g/l |
| L-Tryptophane | 2,00 g/l |
| L-Valine | 11,00 g/l |
| L-Arginine | 24,00 g/l |

3.   Aqueous composition according to claims 1 and 2, characterized in that it addditionally contains further nutrients, such as substitutes for glucose, electrolytes, trace elements, vitamines and/or fats.

**Revendications**

1. Composition aqueuse pour l'alimentation parentérale, contenant les acides aminés essentiels et non essentiels dans un rapport de $N_{essentiel}$ à $N_{non\ essentiel}$ de 1: 1 à 1: 3, caractérisée en ce qu'elle contient comme composant acide aminé uniquement des acides aminés libres contenant des radicaux $\alpha$-amino non substitués en une concentration de 15 à 23% en poids et se compose, pour 100 g, de

| | |
|---|---|
| L-isoleucine | 2,6 — 3,2 g |
| L-leucine | 6,0 — 7,5 g |
| L-lysine | 6,5 — 8,0 g |
| L-méthionine | 2,6 — 3,2 g |
| L-phénylalanine | 4,0 — 5,0 g |
| L-thréonine | 3,0 — 5,0 g |
| L-tryptophane | 0,8 — 1,2 g |
| L-valine | 5,0 — 6,0 g |
| L-arginine | 10,0 — 12,5 g |
| L-histidine | 1,8 — 3,0 g |
| L-alanine | 10,0 — 16,0 g |
| Acide L-aspartique | 5,0 — 6,5 g |
| Acide L-glutamique | 8,5 — 12,0 g |
| Glycine | 11,5 — 14,0 g |
| L-proline | 5,5 — 8,0 g |
| L-sérine | 3,5 — 5,0 g |
| L-tyrosine | 0,2 — 0,4 g |
| L-ornithine | 0,1 — 3,0 g |

2. Composition aqueuse suivant la revendication 1, caractérisée en ce qu'elle présente une concentration en acides aminés de 20% en poids et les acides aminés se composent de

| | |
|---|---|
| L-isoleucine | 5,80 g/l |
| L-leucine | 14,00 g/l |
| L-lysine | 14,00 g/l |
| L-méthionine | 6,00 g/l |
| L-phénylalanine | 9,00 g/l |
| L-thréonine | 7,00 g/l |
| L-tryptophane | 2,00 g/l |
| L-valine | 11,00 g/l |
| L-arginine | 24,00 g/l |
| L-histidine | 4,00 g/l |
| L-alanine | 25,00 g/l |
| Acide L-aspartique | 12,00 g/l |
| Acide L-glutamique | 20,00 g/l |
| Glycine | 25,40 g/l |
| L-proline | 11,80 g/l |
| L-sérine | 8,00 g/l |
| L-tyrosine | 0,40 g/l |
| L-ornithine | 0,60 g/l |

3. Composition aqueuse suivant les revendications 1 et 2, caractérisée en ce qu'elle contient aussi d'autres substances nutritives, comme des substituts du glucose, des électrolytes, des oligo-éléments, des vitamines et/ou de la graisse.